# EUROPÄISCHE PATENTANMELDUNG

(11) **EP 3 991 567 A1**
(43) Veröffentlichungstag der Anmeldung: **04.05.2022**
(21) Anmeldenummer: 20204250.3
(22) Anmeldetag: 28.10.2020
(51) Int. Cl.: A23J 3/04, A23D 9/02, A23J 3/00, A23K 20/147, A23L 33/17, A61K 35/62, A61K 35/64, C11B 1/00, F26B 1/00, F26B 3/092, F26B 9/00, F26B 9/08, F26B 21/00

(54) **VERFAHREN ZUR HERSTELLUNG EINES PROTEINMEHLS AUS INSEKTEN, INSBESONDERE AUS INSEKTENLARVEN UND AUS INSEKTENPUPPEN, ODER AUS WÜRMERN UND TROCKNUNGSVORRICHTUNG ZUR VERWENDUNG IN EI-NEM SOLCHEN VERFAHREN**

(71) Anmelder: Bühler Insect Technology Solutions AG, 9240 Uzwil (CH)
(72) Erfinder: AARTS, Kees Wilhelmus Petrus, 5262 AD Vught (NL); ARSIWALLA, Tarique, 3701 BR Zeist (NL); JANSEN, Maurits Petrus Maria, 4854 CA Bavel (NL)
(74) Vertreter: Vossius & Partner Patentanwälte Rechtsanwälte mbB

(57) **Zusammenfassung**

Offenbart ist ein Verfahren zur Herstellung eines Proteinmehls aus Insekten, insbesondere aus Insektenlarven und aus Insektenpuppen, oder aus Würmern, umfassend die Schritte:
a) Zerkleinern der Insekten oder Würmer zu einem Mus oder Püree;
b) Aufsplitten des Muses oder des Pürees in eine Fettfraktion, in eine Feststoff-enthaltene Fraktion und in eine wässrige Fraktion;
c) Mischen der wässrigen Fraktion und der Feststoffenthaltenen Fraktion zu einer homogenen Masse;
d) Trocknen der homogenen Masse, wobei ein getrocknetes Proteinmehl erhalten wird.

Weiter offenbart ist eine Trocknungsvorrichtung (11) zur Verwendung in einem solchen Verfahren mit Trocknungseinrichtungen (22, 24, 26) und mit einer Einrichtung (14) zum Zerkleinern von Klumpen der homogenen Masse.

## Beschreibung

Die vorliegende Erfindung betrifft ein Verfahren zur Herstellung eines Proteinmehls aus Insekten, insbesondere aus Insektenlarven und aus Insektenpuppen, oder aus Würmern gemäss Anspruch 1 und eine Trocknervorrichtung für ein derartiges Verfahren gemäss Anspruch 11.

Die Erfindung betrifft das Gebiet der Gewinnung von Nährstoffen, Futtermitteln und Nahrungsmitteln von Insekten oder Würmern. Insbesondere stellt die Erfindung ein Verfahren zur Umwandlung von Insekten oder Würmern in Nährstoffströme vor, welche eine Fettfraktion, eine wässrige Fraktion und/oder eine Feststoff-enthaltene Fraktion umfasst. Die wässrige Fraktion und/oder die Feststoff-enthaltene Fraktion enthalten Protein.

In den letzten Jahrzehnten hat das Interesse an der Verwendung von Insekten und Würmern als Lebens- und Futtermittelquelle zugenommen, insbesondere angesichts des Wachstums der Weltbevölkerung und des steigenden Bedarfs an alternativen und nachhaltigen Proteinquellen für die Nutztierindustrie. Da Insekten und Würmer üblicherweise reich an Proteinen und Fetten sind, weisen sie einen relativ hohen Nähr- bzw. Kalorienwert auf und sind dementsprechend besonders gut geeignet für die menschliche Ernährung sowie für die Nutztierzucht.

Im ökonomischen und ökologischen Sinne ist es wünschenswert, Insekten und Würmer im industriellen Massstab herzustellen und verarbeiten zu können, um standardisiert Nährstoffe zu produzieren, die anschliessend zur Herstellung von Lebens- oder Futtermitteln verwendet werden können.

Die GB 2 239 655 A aus dem artfremden Gebiet der Verarbeitung von Fischabfällen offenbart ein Verfahren zur Gewinnung von Fischmehl und Fischöl. Die Fischabfälle werden dabei gekocht und anschliessend in Feststoffe und eine Mixtur aus Wasser sowie Reststoffen aufgeteilt. Die Mixtur wird erwärmt und dann mittels Zentrifugieren separiert. Das Restwasser wird verdampft, um die Feststoffe daraus zu konzentrieren, welche anschliessend getrocknet und gemahlen werden. Als Endprodukt liegen Fischmehl und Fischöl vor.

Nachteilig an diesem bekannten Verfahren ist, dass dieses auf die Verarbeitung von Fischabfällen ausgerichtet ist und unter anderem aufgrund des Kochschritts für eine Verarbeitung von Insekten und Würmern ungeeignet ist. Zudem weist dieses Verfahren zur Gewinnung von Fischmehl und Fischöl aus Fischabfällen einen hohen Energieaufwand auf, was weder nachhaltig noch wirtschaftlich ist.

Aus mehreren Veröffentlichungen ist bekannt, dass bestimmte Nährstoffe von Insekten, wie Proteine oder Fette, gewonnen werden können.

Beispielsweise die CN 104531333 A offenbart ein Verfahren zur Gewinnung von Fett aus bestimmten Insektenlarven. Dabei werden gealterte Insektenlarven oder frische Insektenpuppen bei einer konstanten Temperatur getrocknet. Die getrocknete Masse wird fein vermahlen. Die vermahlene Masse wird mit Aceton oder Gasolin behandelt, dann Überreste herausgefiltert und Fett durch Verdampfen gewonnen.

Nachteilig an diesem bekannten Verfahren ist, dass auch dieses einen hohen Energiebedarf aufweist und chemische Zwischenschritte aufweist, welche für die Gewinnung eines hochwertigen Proteinmehls aus Insekten oder Würmer ungeeignet sind.

Es ist eine Aufgabe der vorliegenden Erfindung, zumindest einen Teil der aus dem Stand der Technik bekannten Nachteile zu überwinden. Insbesondere soll ein hochwertiges Proteinmehl aus Insekten, insbesondere aus Insektenlarven und aus Insektenpuppen, oder aus Würmern gewonnen werden, wobei das Verfahren auch aus energetischer Sicht nachhaltig ist. Weiter ist es eine Aufgabe der vorliegenden Erfindung, eine insbesondere für ein solches Verfahren geeignete Trocknungsvorrichtung zur Verfügung zu stellen. Dabei soll das Verfahren wie auch die Trocknungsvorrichtung des Weiteren für eine industrielle Anwendung geeignet sein.

Diese Aufgaben werden zumindest teilweise durch die Merkmale der unabhängigen Ansprüche gelöst. Weitere vorteilhafte Ausführungen ergeben sich aus Kombinationen mit den Merkmalen der entsprechenden Unteransprüche sowie aus den Ausführungen in der Beschreibung und den Figuren.

Das erfindungsgemässe Verfahren zur Herstellung eines Proteinmehls aus Insekten oder aus Würmern umfasst die folgenden Schritte:
a) Zerkleinern der Insekten oder Würmer zu einem Mus oder Püree;
   Durch das Zerkleinern werden die Insekten oder Würmer in einer Form zur Verfügung gestellt, welche die weitere Verarbeitung und somit die Herstellung des Proteinmehls vereinfachen. Die Insekten oder Würmer sollen genug fein zerkleinert werden, damit das Fett vollständig herausgelöst werden kann. Es hat sich jedoch als vorteilhaft erwiesen, wenn bei der Zerkleinerung der Insekten oder Würmer keine Emulsion entsteht, da eine solche die Separierung von Fett bedeutend erschwert.
b) Aufsplitten des Muses oder des Pürees in eine Fettfraktion, in eine Feststoff-enthaltene Fraktion und in eine wässrige Fraktion;
   Die Feststoff-enthaltene Fraktion wie auch die wässrige Fraktion enthalten Proteine. Die wässrige Fraktion enthält lösliche Komponenten, insbesondere Proteine in löslicher Form. Durch die Aufsplittung des Muses oder des Pürees werden die beiden den wesentlichen Anteil des Proteins enthaltenen Fraktionen separiert und ermöglichen damit eine effiziente und wirtschaftliche Gewinnung des in den Insekten oder Würmern vorhandenen Proteins.
c) Mischen der wässrigen Fraktion und der Feststoff-enthaltenen Fraktion zu einer homogenen Masse;
   Damit werden die jeweils eine massgebliche Menge an Proteinen enthaltenen Fraktionen für die weitere Verarbeitung zusammengeführt.
d) Trocknen der homogenen Masse, wobei ein getrocknetes Proteinmehl erhalten wird.
   Das getrocknete Proteinmehl weist eine hohe Konzentration von Proteinen auf und lässt sich einfach und unkompliziert konfektionieren, lagern sowie weiterverwenden.
Das getrocknete Proteinmehl weist vorteilhaft einen Restwassergehalt von weniger als 15 Gw.-%, vorteilhaft von weniger als 10 Gew.-% auf. Es hat sich gezeigt, dass mit einem Restwassergehalt von weniger als 15 Gw.-%, vorteilhaft von weniger als 10 Gew.-%, das getrocknete Proteinmehl eine Lagerbeständigkeit aufweist, die wesentlich länger als bei einem Proteinmehl mit einem höheren Restwassergehalt ist.

Das erfindungsgemässe Verfahren zeichnet sich durch einen einfachen Aufbau sowie eine kontinuierliche Produktion des herzustellenden Proteinmehls aus. Die Produktion kann automatisiert werden und das Verfahren lässt sich in einen kontinuierlichen Prozessablauf integrieren.

Dieses Verfahren hat sich insbesondere zur Herstellung eines Proteinmehls aus Insektenlarven, z. B. Fliegenlarven oder Mehlwurmlarven, als vorteilhaft erwiesen. Die Insekten, die Insektenlarven, Insektenpuppen oder die Würmer lassen sich auch lebend verarbeiten, wobei diese vorteilhaft vorgängig betäubt oder zumindest inaktiviert werden.

Optional kann das Mus oder Püree - z. B. während oder nach dem Zerkleinern (Schritt a)) - erhitzt werden, wobei die Temperatur vorteilhaft so gewählt wird, dass z. B. etwaig vorhandene Mikroorganismen abgetötet und Enzyme inaktiviert werden. Als vorteilhaft hat sich eine Erhitzungstemperatur von 70° C bis 125° C, weiter vorteilhaft von 80° C bis 100° C, erwiesen. Besonders vorteilhaft ist eine Erhitzungstemperatur von 85° C bis 90° C, welche über einen gewissen Zeitraum von z. B. 5 Sekunden bis 10 Minuten gehalten wird. Damit wird der Energiebedarf für die Erhitzung niedrig gehalten, aber die Abtötung von etwaig vorhandenen Bakterien sichergestellt.

Beispielsweise erfolgt das Erhitzen in einer Zentrifuge, wobei die Haltezeit 15 Sekunden bei 90°C beträgt. In der Zentrifuge verbleibt das Mus oder Püree etwa 45 Sekunden und hat eine Austrittstemperatur von etwa 80°C.

Vorzugsweise wird vor dem Schritt c) der Wassergehalt der wässerigen Fraktion reduziert, womit die durch das Mischen gewonnene homogene Masse mit einem geringeren Energieaufwand und ggf. geringeren Zeitaufwand als im unreduzierten Zustand trocknenbar ist (im Schritt d)).

Bevorzugt erfolgt die Reduktion des Wassergehalts der wässrigen Fraktion vor dem Schritt c) in einem Verdampfer, womit sich die Reduzierung des Wassergehalts einfach und energieeffizient durchführen lässt. Der Verdampfer umfasst bspw. einen Evaporator, insbesondere einen Rising Film Evaporator (auch Steigrohr- oder Kletterfilmverdampfer genannt), oder einen Fallstromverdampfer.

Alternativ zu einem Verdampfer kann auch ein Filter eingesetzt werden. Mittels Filterprozesse wird die wässrige Fraktion aufkonzentriert bzw. vorentwässert und somit der Wassergehalts der wässrigen Fraktion reduziert. Für die Filterung hat sich ein Membranfilter als besonders vorteilhaft erwiesen.

Vorzugsweise wird nach dem Schritt d) das getrocknete Proteinmehl vermahlen, womit dieses einfach konfektionierbar und weiterverwendbar ist. Durch das Vermahlen weist das getrocknete Proteinmehl eine grosse Oberfläche auf, was sich bei der Weiterverwendung des Proteinmehls insbesondere in Mischungen als vorteilhaft gezeigt hat.

Bevorzugt erfolgt das Zerkleinern (Schritt a)) in einem Cutter oder in einer Presse, womit das erzeugte Mus oder Püree eine für die Weiterverarbeitung optimale Konsistenz aufweist.

Unter einem "Cutter" wird im vorliegenden Zusammenhang eine Maschine für die Zerkleinerung verstanden, die kontinuierlich betrieben wird und mit einer Schneidvorrichtung(z. B. Messer) ausgestattet ist.

Auch mittels einer Presse lässt sich das Zerkleinern der Larven oder Würmer erzielen, wobei gleichzeitig die Feststoff-enthaltene Fraktion von der Fettfraktion bzw. der wässrigen Fraktion abgeschieden wird.

Vorzugsweise erfolgt das Aufsplitten des Muses oder des Pürees(Schritt b)) in einem Separator, der eine einfache physikalische/mechanische Auftrennung der Fraktionen ermöglicht. Dabei hat sich für diesen Zweck ein, vorteilhaft horizontaler, Mehrphasen-Dekanter als besonders vorteilhaft erwiesen.

Wird das Mus bzw. das Püree mittels eines Cutters oder dergleichen zerkleinert, erfolgt die Aufsplittung vorteilhaft in einem 3-Phasen-Separator, so dass anschliessend die Fettfraktion, die Feststoff-enthaltene Fraktion und die wässrige Fraktion zur weiteren Verwendung vorliegt.

Wird das Mus bzw. das Püree mittels einer Presse oder dergleichen zerkleinert, erfolgt die Aufsplittung vorteilhaft in einem 2-Phasen-Separator, da die Feststoff-enthaltende Fraktion bereits beim Pressen abgeschieden wird. Im 2-Phasen-Separator werden dann die Fettfraktion und die wässrige Fraktion abgeschieden. Anschliessend liegt ebenfalls die Fettfraktion, die Feststoff-enthaltene Fraktion und die wässrige Fraktion vor.

Bevorzugt werden Klumpen der homogenen Masse im Schritt c) und/oder im Schritt d) zerkleinert (= Schritt e)), womit das getrocknete Proteinmehl eine gleichmässigere Partikelgrösse aufweist.

Das Zerkleinern der Klumpen kann beispielsweise in dem Mischer vor, während oder nach dem Mischvorgang (Schritt c)) erfolgen. Der Mischer weist dazu z. B. eine entsprechende Einrichtung auf, wie beispielsweise einen sogenannten "Lump Breaker".

Alternativ oder ergänzend erfolgt das Zerkleinern der Klumpen beispielsweise beim Trocknen (Schritt d)). Dafür ist beispielsweise eine entsprechende Einrichtung vorgesehen, welche Bestandteil einer Trocknungsvorrichtung sein kann.

Vorzugsweise wird die homogene Masse während dem Mischen (Schritt c)) mittels einer Fördereinrichtung zum Trocknen mittels einer Trocknungsvorrichtung (Schritt d)) zugeführt, um eine unerwünschte Ansammlung der homogenen Masse in der Trocknungsvorrichtung zu vermeiden, da eine solche Ansammlung eine gleichmässige Trocknung der homogenen Masse stören kann. Die homogene Masse wird mittels der Fördereinrichtung vorteilhaft kontinuierlichen eingebracht.

Alternativ oder ergänzend wird die homogene Masse nach dem Mischen (Schritt c)) mittels zumindest einer Fördereinrichtung zum Trocknen mittels einer Trocknungsvorrichtung (Schritt d)) zugeführt.

Als weiter vorteilhaft hat sich eine Ausgestaltung erwiesen, bei der die zumindest eine Fördereinrichtung Bestandteil eines Mischers ist.

Es können zudem mehrere, gegebenenfalls unterschiedlich ausgestaltete Fördereinrichtungen vorgesehen sein, welche parallel oder seriell zueinander angeordnet sind.

Optional werden Anteile des getrockneten Produkts dem Mischer vor oder während dem Mischen (im Schritt c)) zugeführt, womit sich in den zu mischenden Fraktionen befindliche Wasseranteile binden lassen. Die daraus resultierende homogene Masse weist damit einen niedrigeren Wassergehalt auf.

Vorzugsweise wird das getrocknete Produkt nach dem Trocknen (Schritt d)) oder nach dem Vermahlen (Schritt e)) gekühlt, was die Weiterverarbeitung und Weiterverwendung beschleunigt. Zudem lässt sich ein gekühltes Proteinmehl einfach konfektionieren, z. B. in Säcke abfüllen, da sich durch die Kühlung das Risiko der Kondensation von Luftfeuchtigkeit am warmen Produkt verringert.

In einem weiteren Aspekt betrifft die Erfindung auch eine Trocknungsvorrichtung zur Verwendung zumindest in einem Teil des vorgenannten Verfahrens, wobei die Trocknungsvorrichtung zumindest eine Trocknungseinrichtung und eine Einrichtung zum Zerkleinern von Klumpen der homogenen Masse aufweist.

Die zumindest eine Trocknungseinrichtung umfasst beispielsweise eine thermische Quelle und/oder eine Strahlungsquelle zum Trocknen der homogenen Masse.

Die Einrichtung zum Zerkleinern von Klumpen der homogenen Masse kann auf die homogene Masse einwirkende Elemente aufweisen oder eine spezielle Betriebsart, beispielsweise der Trocknungsvorrichtung selbst oder der Teile davon, sein.

Vorzugsweise ist diese Trocknungsvorrichtung als Fliessbetttrockner ausgebildet, wobei die Bewegung des Fliessbetts und somit die spezielle Betriebsart des Fliessbetttrockners die Einrichtung zum Zerkleinern von Klumpen der homogenen Masse bildet.

Beispielsweise kommt ein Vibrations-Fliessbetttrockner ("Vibrating Fluidized Bed Dryer") zur Anwendung, welcher durch die Bewegung des Fliessbetts ein einfaches Zerkleinern von Klumpen der homogenen Masse gewährleistet.

Als besonders vorteilhaft für das Zerkleinern von Klumpen der homogenen Masse hat sich ein schüttelnder Fliessbetttrockner ("Shaking Fluidized Bed Dryer") erwiesen, bei dem sich nicht nur das Fliessbett bewegt, sondern der gesamte Fliessbetttrockner schüttelt. Im Gegensatz zu einem Vibrations-Fliessbetttrockner ist ein schüttelnder Fliessbetttrockner besser für klebrige Materialien geeignet. Des Weiteren ist der Transport der homogenen Masse beziehungsweise des Proteinmehls durch die Trocknungsvorrichtung verbessert, infolge einer Art von Wurfbewegung, welche sich auf dem Fliessbett ergibt. Zudem erfolgt aufgrund dieser Art der Bewegung des Fliessbetts eine Trocknung nach dem Prinzip "First-In"/"First-Out", so die in die Trocknungsvorrichtung eingebrachten Anteile der zu trocknenden homogenen Masse gleichmässig sowie kontinuierlich und somit nicht statisch getrocknet werden.

Als sehr vorteilhaft hat sich eine Bewegung des Fliessbetts bzw. des Fliessbetttrockners mit einer tiefen Frequenz und einer hohen Amplitude erwiesen. Die Bewegungsfrequenz (auch Schüttelfrequenz genannt) des Fliessbetts bzw. des schüttelnden Fliessbetttrockners liegt vorteilhaft bei 2.5 Hz bis 4.0 Hz. Idealweise liegt die Bewegungsfrequenz bei 3.0 Hz bis 3.5 Hz und besonders bevorzugt bei 3.3 Hz bis 3.4 Hz.

Alternativ ist die Trocknungsvorrichtung als Rotationstrockner ausgebildet. Unter einem "Rotationstrockner" werden in diesem Zusammenhang jegliche Trocknungsvorrichtungen verstanden, die eine rotierende, vorteilhaft mechanische Vorrichtung zur Zerkleinerung von Klumpen aufweisen. Als nicht abschliessende Beispiele seien hier Trockner mit einem Mahlrotor-System, Paddle Dryer, Turbo Dryer, etc. genannt.

Bevorzugt ist die zugeführte Trocknungslufttemperatur kleiner gleich 200° C, womit eine ausreichende starke Trocknung der homogenen Masse ermöglicht ist. Als besonders bevorzugt hat sich eine Trocknungslufttemperatur erwiesen, die im Bereich von 110° C bis 160° C liegt. Trocknungslufttemperaturen in den vorgenannten Temperaturbereichen gewährleisten eine ausreichende Trocknung der homogenen Masse ohne die Qualität derselben massgeblich zu reduzieren. Die Temperatur der homogenen Masse wird bei dem Trocknungsvorgang vorteilhaft kleiner als 100° C und besonders bevorzugt kleiner als 70° C gehalten.

Vorzugsweise sind zumindest zwei Behandlungszonen vorgesehen, was eine vorteilhafte Behandlung der homogenen Masse in der Trocknungsvorrichtung ermöglicht.

Besonders vorteilhaft hat sich eine Trocknungsvorrichtung erwiesen, die drei Behandlungszonen aufweist, nämlich eine erste Behandlungszone, eine zweite Behandlungszone und eine dritte Behandlungszone. Beispielsweise wird in der ersten Behandlungszone (Trocknungszone) die der Trocknungsvorrichtung zugeführte homogene Masse vorgetrocknet, wobei vorteilhaft hier insbesondere der Wassergehalt der zugeführten homogenen Masse wesentlich reduziert wird. In der zweiten Behandlungszone (Trocknungszone) findet beispielsweise die Haupttrocknung der zugeführten homogenen Masse statt, in welcher der Wassergehalt der homogenen Masse auf das gewünschte Mass reduziert wird. Die dritte Behandlungszone (Kühlungszone) dient beispielsweise der Kühlung des getrockneten Proteinmehls, womit dieses einfacher händelbar, z. B. verpackbar, ist.

Weiterhin ist es auch denkbar, dass die Trocknervorrichtung mehrere weitere Behandlungszonen aufweist. Auf diese Weise kann unter Umständen eine noch effektivere bzw. gezieltere Trocknung der homogenen Masse erreicht und auf besondere Eigenschaften der homogenen Masse bzw. des herzustellenden Proteinmehls Rücksicht genommen werden.

Alternativ oder ergänzend können auch zwei oder mehrere Trocknungsvorrichtungen hintereinander angeordnet werden, so dass zur Trocknung und/oder Kühlung dadurch mehrere Behandlungszonen zur Verfügung stehen.

Im Folgenden wird die Erfindung anhand eines Ausführungsbeispiels und mehrerer Zeichnungen näher erläutert. Dabei zeigen
- Figur 1:: eine schematische Darstellung eines erfindungsgemässen Verfahrens;
- Figur 2:: eine schematische Darstellung eines weiteren erfindungsgemässen Verfahrens;
- Figur 3:: eine erfindungsgemässe Trocknervorrichtung in einer schematischen Seitenansicht;
- Figur 4:: eine Variante der in Fig. 3 gezeigten Trocknungsvorrichtung in einem Teilschnitt;
- Figur 5:: eine weitere Variante der in Fig. 3 gezeigten Trocknungsvorrichtung in einem Teilschnitt.

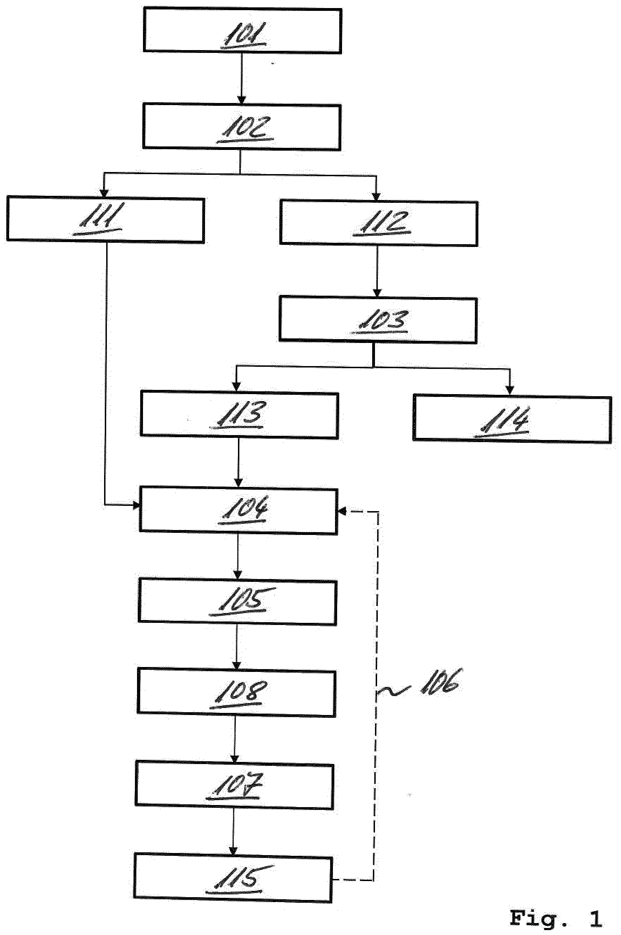

Gemäss dem in der Figur 1 gezeigten Verfahren zur Herstellung eines Proteinmehls aus Insektenlarven werden zuerst die Insektenlarven (hier Larven der Black Soldier Fly (BSF)) zugeführt (Schritt 101). In einer Presse werden die Insektenlarven zu einem Mus oder Püree zerkleinert (Schritt 102), wobei eine Feststoff-enthaltene Fraktion 111 von einer flüssigen Fraktion 112 abgeschieden wird.

Diese flüssige Fraktion 112 wird in einem 2-Phasen-Spearator in eine wässrige Fraktion 113 und eine Fettfraktion 114 aufgesplittet. Der Wassergehalt der wässerigen Fraktion 113 kann vor der weiteren Verarbeitung reduziert werden, z. B. in einem Verdampfer oder in einem Membranfilter.

Die Feststoff-enthaltene Fraktion 111 und die wässrige Fraktion 113, welche beide Protein enthalten, werden in einem Mischer zu einer homogenen Masse gemischt (Schritt 104). Etwaige Klumpen der homogenen Masse werden während dem Mischen zerkleinert. Zur Reduktion des Wassergehalts der homogenen Masse werden Anteile des getrockneten Proteinmehls dem Mischer während dem Mischen zugeführt (Schritt 106). Optional wird die homogene Masse nach dem Mischen gekühlt.

Der Mischer weist beispielsweise eine Fördereinrichtung auf, mittels der die gemischte homogene Masse der Trocknungsvorrichtung zum Trocknen zugeführt wird. Die Fördereinrichtung ist z. B. Bestandteil des Mischers.

Die homogene Masse wird anschliessend in einem schüttelnden Fliessbetttrockner als Trocknungsvorrichtung getrocknet (Schritt 105), bis das getrocknete Proteinmehl 115 vorliegt. Etwaige Klumpen der homogenen Masse werden während dem Trocknen zerkleinert. Nach oder während dem Trocknen (Schritt 105) kann das getrocknete Proteinmehl 115 vermahlen werden (Schritt 107). Das getrocknete Proteinmehl 115 wird nach dem Trocknen (Schritt 105) gekühlt (= Schritt 108).

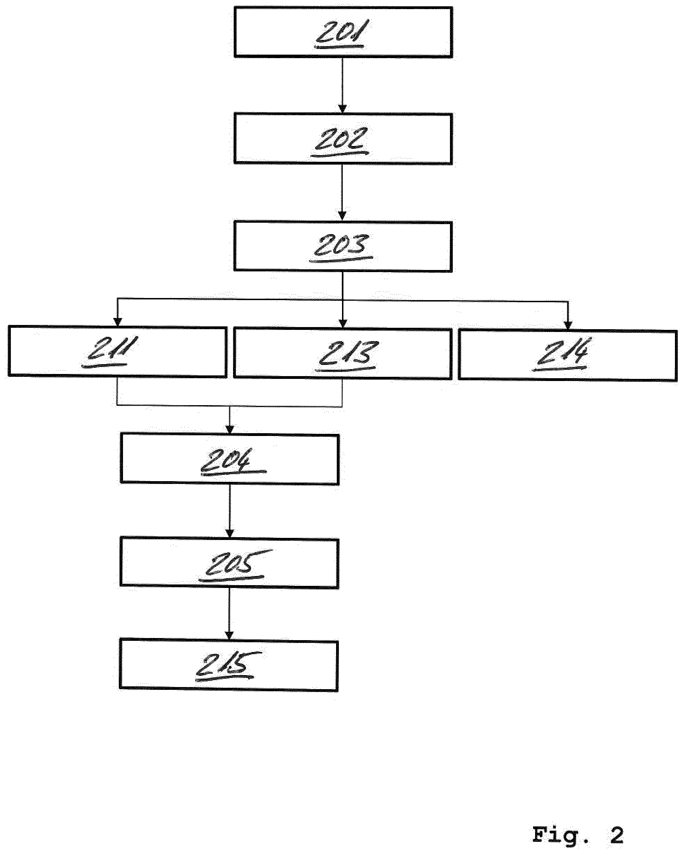

Gemäss dem in der Figur 2 gezeigten alternativen Verfahren zur Herstellung eines Proteinmehls aus Insektenlarven werden zuerst die Insektenlarven (hier Larven der Black Soldier Fly (BSF)) einem Cutter zugeführt (Schritt 201). In dem Cutter werden die Insektenlarven zu einem Mus oder Püree zerkleinert (Schritt 202).

Dieses Mus oder Püree wird in einem 3-Phasen-Sparator (Schritt 203) in eine Feststoff-enthaltene Fraktion 211, eine wässrige Fraktion 213 und eine Fettfraktion 214 aufgesplittet. Der Wassergehalt der wässerigen Fraktion 213 kann vor der weiteren Verarbeitung reduziert werden, z. B. in einem Verdampfer oder in einem Membranfilter.

Die Feststoff-enthaltene Fraktion 211 und die wässrige Fraktion 213, welche beide Protein enthalten, werden in einem Mischer zu einer homogenen Masse gemischt (Schritt 204). Etwaige Klumpen der homogenen Masse werden während dem Mischen zerkleinert. Zur Reduktion des Wassergehalts der homogenen Masse können Anteile des getrockneten Proteinmehls 215 dem Mischer während dem Mischen zugeführt.

Die homogene Masse wird anschliessend in einem Rotationsmischer als Trocknungsvorrichtung getrocknet (Schritt 205), bis das getrocknete Proteinmehl 215 vorliegt.

Beispielsweise in Abhängigkeit der weiteren Verwendung kann das getrocknete Proteinmehl 215 anschliessend vermahlen werden. Alternativ oder ergänzend kann das getrocknete Proteinmehl 215 auch gekühlt werden.

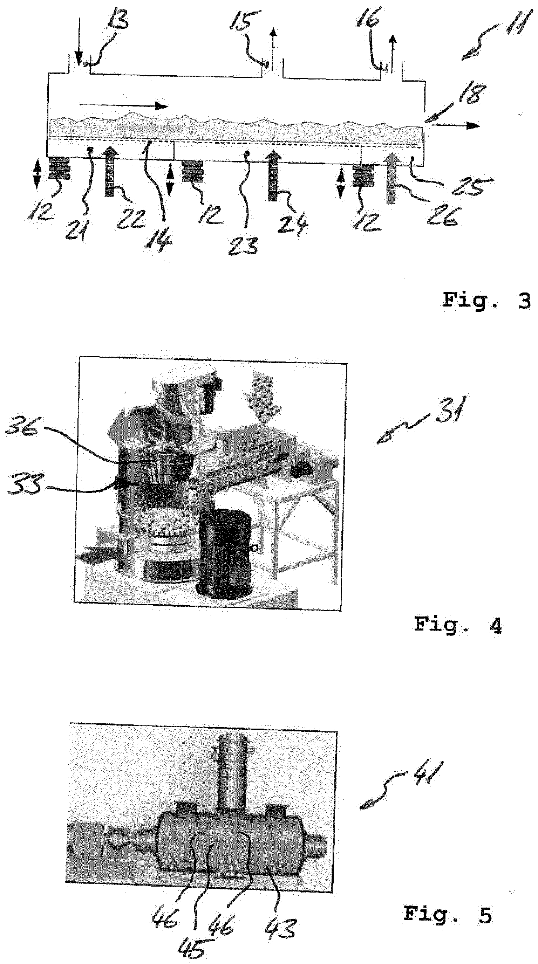

Die in der Figur 3 gezeigte Trocknungsvorrichtung 11 ist als schüttelnder Fliessbetttrockner ausgebildet, der auf Lagern 12 gelagert ist. Mittels der Lager 12 wird die Trocknungsvorrichtung 11 auch geführt, z. B. in Richtung der Doppelpfeile. Die Bewegungsfrequenz der Trocknungsvorrichtung 11 liegt bei 2.5 Hz bis 4.0 Hz, idealerweise bei 3.0 Hz bis 3.5 Hz, bevorzugt bei 3.3 Hz bis 3.4 Hz.

Die Trocknungsvorrichtung 11 weist als Trocknungseinrichtungen eine erste Zuführung 22 für heisse Luft mit einer Lufttemperatur von 140° C bis 150° C und eine zweite Zuführung 24 für heisse Luft mit einer Lufttemperatur von 150° C bis 180° C beziehungsweise kleiner 200° C sowie eine dritte Zuführung 26 für gekühlte Luft oder Umgebungsluft mit einer Lufttemperatur von 15° C bis 25° C auf. Die erste Zuführung 22 ist in einer ersten Behandlungszone 21, die zweite Zuführung 24 in einer zweiten Behandlungszone 23 und die dritte Zuführung 26 in einer dritten Behandlungszone 25 vorgesehen.

Die Trocknungsvorrichtung 11 weist eine Eintrittsöffnung 13 für die homogene Masse auf, welche nach dem Zuführen in die Trocknungsvorrichtung 11 auf dem Fliessbett 14 zu liegen kommt. Weiter sind zwei Abluftöffnungen 15 und 16 vorgesehen, durch welche Luft aus der Trocknungsvorrichtung 11 austreten kann.

Die auf dem Fliessbett 14 zu liegen kommende homogene Masse wird durch die Bewegung des Fliessbetts 14, welche sich aufgrund des im Betrieb schüttelnden Fliessbetttrockners ergibt, kontinuierlich von der Eintrittsöffnung 13 zu einer Austrittsöffnung 18 transportiert, aus welcher das getrocknete Proteinmehl austritt. Während des Transports auf dem Fliessbett 14 durchläuft die homogene Masse die erste Behandlungszone 21, in welcher die homogene Masse vorgetrocknet wird, die zweite Behandlungszone 23, in welcher die homogene Masse zum Proteinmehl getrocknet wird, und die dritte Behandlungszone 25, in welcher das getrocknete Proteinmehl gekühlt wird.

Das sich bewegende Fliessbett 14 bildet die Einrichtung zum Zerkleinern von Klumpen der homogenen Masse der Trocknungsvorrichtung 11.

In Figur 4 ist eine Trocknungsvorrichtung 31 gezeigt, die als Rotationstrockner ausgebildet ist. Die Trocknungsvorrichtung 31 weist als Einrichtung zur Zerkleinerung von Klumpen in der homogenen Masse ein Mahlrotorsystem 36 im Trocknungsraum 33 auf. Der Trocknungsraum 33 bildet die Trocknungseinrichtung der Trocknungsvorrichtung 31. Die homogene Masse wird während dem Trocknen vermahlen und dabei werden Klumpen, die in der homogenen Masse vorhanden sind oder die beim Trocknungsvorgang entstehen, zerstört. Die Trocknungstemperatur im Trocknungsraum 33 beträgt weniger als 200° C. Der Trocknungsraum 33 kann beispielsweise unterteilt sein und in diesen Unterteilungen unterschiedliche Temperaturen aufweisen, so dass die Trocknungsvorrichtung 31 mehrere Behandlungszonen aufweist.

Anstelle eines Mahlrotorsystems 36 kann ein rotierender Mischer (hier nicht dargestellt) vorgesehen sein, der eine Zerstörung von Klumpen, die in der homogenen Masse vorhanden sind oder beim Trocknungsvorgang entstehen, einfach ermöglicht. Je nach Art der Zusammensetzung und/oder der Konsistenz der homogenen Masse wird eine vorteilhafte Geometrie des rotierenden Mischers gewählt.

Figur 5 zeigt eine Trocknungsvorrichtung 41 gezeigt, die ebenfalls als Rotationstrockner ausgebildet ist, nämlich als so genannter Turbo Dryer. Ein Trocknungsraum 43 bildet die Trocknungseinrichtung der Trocknungsvorrichtung 41. Im Trocknungsraum 43 ist ein schnell rotierender Rotor 45 mit Flügeln 46 vorgesehen, welcher die homogene Masse während dem Trocknen mit hoher Geschwindigkeit gegen die Wand des Trocknungsraums 43 schmeisst, wobei Klumpen, die in der homogene Masse vorhanden sind oder die beim Trocknungsvorgang entstehen, zerstört werden. Die Trocknungstemperatur im Trocknungsraum 43 beträgt weniger als 200° C. Der Trocknungsraum 43 kann ebenfalls unterteilt sein und in diesen Unterteilungen unterschiedliche Temperaturen aufweisen, so dass die Trocknungsvorrichtung 41 mehrere Behandlungszonen aufweist.

### Bezugszeichenliste:

- 11: Trocknungsvorrichtung
- 12: Lager
- 13: Eintrittsöffnung
- 14: Fliessbett
- 15: 1. Auslassöffnung
- 16: 2. Auslassöffnung

- 18: Austrittsöffnung

- 21: 1. Behandlungszone
- 22: 1. Zuführung
- 23: 2. Behandlungszone
- 24: 2. Zuführung
- 25: 3. Behandlungszone
- 26: 3. Zuführung

- 31: Trocknungsvorrichtung
- 33: Trocknungsraum
- 36: Mahlrotorsystem

- 41: Trocknungsvorrichtung
- 43: Trocknungsraum
- 45: Rotor
- 46: Flügel

- 101: Zuführen Insektenlarven
- 102: Zerkleinern der Insektenlarven
- 103: Separieren des Mus oder Pürees
- 104: Mischen v. 111 und 113
- 105: Trocknen
- 106: Zuführen von 115 an 104
- 107: Vermahlen
- 108: Kühlen
- 111: Feststoff-enthaltene Fraktion
- 112: flüssige Fraktion
- 113: wässrige Fraktion
- 114: Fettfraktion
- 115: getrocknetes Proteinmehl

- 201: Zuführen Insektenlarven
- 202: Zerkleinern der Insektenlarven
- 203: Separieren des Mus oder Pürees
- 204: Mischen v. 211 und 213
- 205: Trocknen

- 211: Feststoff-enthaltene Fraktion

- 213: wässrige Fraktion
- 214: Fettfraktion
- 215: getrocknetes Proteinmehl

## Patentansprüche

1. Verfahren zur Herstellung eines Proteinmehls (115; 215) aus Insekten, insbesondere aus Insektenlarven und aus Insektenpuppen, oder aus Würmern, umfassend die Schritte:
a) Zerkleinern (102; 202) der Insekten oder Würmer zu einem Mus oder Püree;
b) Aufsplitten (103; 203) des Muses oder des Pürees in eine Fettfraktion (114; 214), in eine Feststoff-enthaltene Fraktion (111; 211) und in eine wässrige Fraktion (113; 213);
c) Mischen (104; 204) der wässrigen Fraktion (113; 213) und der Feststoff-enthaltenen Fraktion (111; 211) zu einer homogenen Masse;
d) Trocknen (105; 205) der homogenen Masse, wobei ein getrocknetes Proteinmehl (115; 215) erhalten wird.

2. Verfahren gemäss Anspruch 1,
wobei vor dem Schritt c) (104; 204) der Wassergehalt der wässerigen Fraktion (113; 213) reduziert wird.

3. Verfahren gemäss Anspruch 2,
wobei die Reduktion des Wassergehalts der wässrigen Fraktion (113; 213) in einem Verdampfer oder in einem Filter, vorteilhaft in einem Membranfilter, erfolgt.

4. Verfahren gemäss einem der vorangehenden Ansprüche,
wobei nach dem Schritt d) (105; 205) das getrocknete Proteinmehl (115; 215) vermahlen wird (= Schritt 107).

5. Verfahren gemäss einem der vorangehenden Ansprüche,
wobei das Zerkleinern (Schritt a); 102;202) in einem Cutter oder in einer Presse erfolgt.

6. Verfahren gemäss einem der vorangehenden Ansprüche,
wobei das Aufsplitten (103; 203) des Muses oder des Pürees gemäss Schritt b) in einem Separator, vorteilhaft in einem 2- oder 3-Phasen-Separator, erfolgt.

7. Verfahren gemäss einem der vorangehenden Ansprüche,
ferner mit dem Schritt e) zum Zerkleinern von Klumpen der homogenen Masse im Schritt c) (104; 204) oder im Schritt d) (105; 205).

8. Verfahren gemäss einem der vorangehenden Ansprüche,
wobei die homogene Masse während und/oder nach dem Schritt c) (104; 204) mittels zumindest einer Fördereinrichtung zum Trocknen der Trocknungsvorrichtung zugeführt wird, wobei vorteilhaft die zumindest eine Fördereinrichtung Bestandteil eines Mischers ist.

9. Verfahren gemäss einem der vorangehenden Ansprüche,
wobei Anteile des getrockneten Proteinmehls (115; 215) dem Mischer (im Schritt c); 104; 204) zugeführt werden.

10. Verfahren gemäss einem der vorangehenden Ansprüche, wobei das getrocknete Proteinmehls (115) nach dem Schritt d) (105) oder nach dem Schritt e) (107) gekühlt wird.

11. Trocknungsvorrichtung (11; 31; 41) zur Verwendung in einem Verfahren gemäss einem der vorangehenden Ansprüche, mit zumindest einer Trocknungseinrichtung (22, 24, 26; 33; 43), **dadurch gekennzeichnet,**
**dass** eine Einrichtung (14; 36; 45, 46) zum Zerkleinern von Klumpen der homogenen Masse vorgesehen ist.

12. Trocknungsvorrichtung gemäss Anspruch 11, ausgebildet als Fliessbetttrockner (11), vorzugsweise als geschüttelter Fliessbetttrockner mit einer Bewegungsfrequenz vorteilhaft bei 2.5 Hz bis 4.0 Hz, idealerweise bei 3.0 Hz bis 3.5 Hz, bevorzugt bei 3.3 Hz bis 3.4 Hz.

13. Trocknungsvorrichtung gemäss Anspruch 11, ausgebildet als Rotationstrockner (31; 41).

14. Trocknungsvorrichtung einem der Ansprüche 11 bis 13, wobei die zugeführte Trocknungslufttemperatur kleiner gleich 200° C, vorzugsweise 110° C bis 160° C, ist.

15. Trocknungsvorrichtung einem der Ansprüche 11 bis 14, wobei zumindest zwei Behandlungszonen (21, 23, 25) vorgesehen sind.

## Geänderte Patentansprüche

### Geänderte Patentansprüche gemäss Regel 137(2) EPÜ.

1. Verfahren zur Herstellung eines Proteinmehls (115; 215) aus Insekten, insbesondere aus Insektenlarven und aus Insektenpuppen, oder aus Würmern, umfassend die Schritte:
a) Zerkleinern (102; 202) der Insekten oder Würmer zu einem Mus oder Püree;
b) Aufsplitten (103; 203) des Muses oder des Pürees in eine Fettfraktion (114; 214), in eine Feststoffenthaltene Fraktion (111; 211) und in eine wässrige Fraktion (113; 213);
b1) Reduzieren des Wassergehalts der wässrigen Fraktion (113; 213);
c) Mischen (104; 204) der reduzierten wässrigen Fraktion (113; 213) und der Feststoff-enthaltenen Fraktion (111; 211) zu einer homogenen Masse;
d) Trocknen (105; 205) der homogenen Masse, wobei ein getrocknetes Proteinmehl (115; 215) erhalten wird.

2. Verfahren gemäss Anspruch 2,
wobei die Reduktion des Wassergehalts der wässrigen Fraktion (113; 213) in einem Verdampfer oder in einem Filter, vorteilhaft in einem Membranfilter, erfolgt.

3. Verfahren gemäss einem der vorangehenden Ansprüche, wobei nach dem Schritt d) (105; 205) das getrocknete Proteinmehl (115; 215) vermahlen wird (= Schritt 107).

4. Verfahren gemäss einem der vorangehenden Ansprüche, wobei das Zerkleinern (Schritt a); 102;202) in einem Cutter oder in einer Presse erfolgt.

5. Verfahren gemäss einem der vorangehenden Ansprüche, wobei das Aufsplitten (103; 203) des Muses oder des Pürees gemäss Schritt b) in einem Separator, vorteilhaft in einem 2- oder 3-Phasen-Separator, erfolgt.

6. Verfahren gemäss einem der vorangehenden Ansprüche, ferner mit dem Schritt e) zum Zerkleinern von Klumpen der homogenen Masse im Schritt c) (104; 204) oder im Schritt d) (105; 205).

7. Verfahren gemäss einem der vorangehenden Ansprüche, wobei die homogene Masse während und/oder nach dem Schritt c) (104; 204) mittels zumindest einer Fördereinrichtung zum Trocknen der Trocknungsvorrichtung zugeführt wird, wobei vorteilhaft die zumindest eine Fördereinrichtung Bestandteil eines Mischers ist.

8. Verfahren gemäss einem der vorangehenden Ansprüche, wobei Anteile des getrockneten Proteinmehls (115; 215) dem Mischer (im Schritt c); 104; 204) zugeführt werden.

9. Verfahren gemäss einem der vorangehenden Ansprüche, wobei das getrocknete Proteinmehls (115) nach dem Schritt d) (105) oder nach dem Schritt e) (107) gekühlt wird.

10. Trocknungsvorrichtung (11; 31; 41), wobei die Trocknungsvorrichtung (11; 31; 41) in einem Verfahren gemäss einem der vorangehenden Ansprüche verwendet wird, mit zumindest einer Trocknungseinrichtung (22, 24, 26; 33; 43), **dadurch gekennzeichnet,**
**dass** eine Einrichtung (14; 36; 45, 46) zum Zerkleinern von Klumpen der homogenen Masse vorgesehen ist.

11. Trocknungsvorrichtung gemäss Anspruch 10, ausgebildet als Fliessbetttrockner (11), vorzugsweise als geschüttelter Fliessbetttrockner, der konfiguriert ist, mit einer Bewegungsfrequenz vorteilhaft bei 2.5 Hz bis 4.0 Hz, idealerweise bei 3.0 Hz bis 3.5 Hz, bevorzugt bei 3.3 Hz bis 3.4 Hz zu arbeiten.

12. Trocknungsvorrichtung gemäss Anspruch 10, ausgebildet als Rotationstrockner (31; 41).

13. Trocknungsvorrichtung einem der Ansprüche 10 bis 12, wobei die Trocknungsvorrichtung konfiguriert ist, zugeführte Trocknungslufttemperatur kleiner gleich 200° C, vorzugsweise 110° C bis 160° C, bereitzustellen.

14. Trocknungsvorrichtung einem der Ansprüche 10 bis 13, wobei zumindest zwei Behandlungszonen (21, 23, 25) vorgesehen sind.
